# EUROPEAN PATENT APPLICATION

(11) **EP 3 552 657 A1**
(43) Date of publication of application: **16.10.2019**
(21) Application number: 16920956.6
(22) Date of filing: 11.11.2016
(51) Int. Cl.: A61M 35/00, A61N 1/30, A61N 1/32, A61H 15/02, A61M 37/00

(54) **CONTAINER HAVING APPLICATION PART**

(71) Applicant: Chong Woo Co., Ltd., Incheon 22756 (KR)
(72) Inventor: LEE, Chung Kee, Seoul 08011 (KR)
(74) Representative: Stona, Daniel
(86) International application number: PCT/KR2016/012965
(87) International publication number: WO 2018/088587

(57) **Abstract**

The present invention provides a container having an application part, which comprises a composition holding part and a composition application part, wherein the application part is equipped with two or more rotating bodies, and as the material of the rotating bodies, a metal is selected that forms a potential difference due to an ionization phenomenon of a liquid at a part being used and/or the composition contained in the holding part.

## Description

### [Technical Field]

The present invention relates to a container having an application part. More particularly, the present invention relates to a container having an application part provided with a rotary body.

### [Background Art]

Conventionally, various containers having rotary bodies have been widely used. In the case of such conventional containers, application is easily performed, and at the same time an acupressure effect is achieved by the pressure and vibration of the rotary bodies during application (Patent Document 1).

Meanwhile, a cosmetic product having a rotary body made of different kinds of materials is disclosed (Patent Document 2). In the case of this cosmetic product, a liquid is ionized. When this cosmetic product is used for dry skin, therefore, ionization of the liquid is not expected, and the skin is rather burdened.

### [Prior Art Document]

### [Patent Document]

Patent Document 1: Japanese Utility Model Application Publication No. M 50-39196
Patent Document 2: Japanese Patent Application Publication No. H 7-96019

### [Disclosure]

### [Technical Problem]

Therefore, the present invention has been made in view of the above problems, and it is an object of the present invention to provide a container having an application part that is capable of generating a galvanic current between different kinds of metals through a simple structure in order to achieve high cosmetic effects. It is another object of the present invention to provide a product that is capable of being easily used not only on wrinkles of a face but also on wide regions of the face such as the cheeks.

### [Technical Solution]

In accordance with a first aspect of the present invention, the above objects can be accomplished by the provision of a container including a composition reception part and a composition application part, wherein the composition application part is provided with two or more rotary bodies, and each of the rotary bodies is made of metals having a potential difference therebetween caused by the ionization of a composition received in the composition reception part and/or a liquid in the region at which each of the rotary bodies is used.

In accordance with a second aspect of the present invention, the above object can be accomplished by the provision of a container including a composition reception part and a composition application part, wherein the composition application part is provided with a single rotary body, and the rotary body has two or more metal portions having a potential difference therebetween caused by the ionization of a composition received in the composition reception part and/or a liquid in the region at which the rotary body is used.

In accordance with a third aspect of the present invention, the above object can be accomplished by specifying the metal or the material for each of the metal portions.

In accordance with a fourth aspect of the present invention, the above object can be accomplished by configuring the composition application part so as to be rotatable.

According to the present invention, it is possible to generate a galvanic current through a simple structure, and it is also possible to provide high cosmetic effects. Furthermore, it is possible to provide a container that is capable of being easily used not only on wrinkles of a face but also on wide regions of the face such as the cheeks through the use of a composition application part, which is configured to be rotatable.

### [Description of Drawings]

FIG. 1 is a side view showing a container according to the present invention having two balls as rotary bodies;
FIG. 2 is an enlarged sectional view showing an application part of the container having two balls as the rotary bodies;
FIG. 3 is a front view showing the application part of the container having two balls as the rotary bodies in the state in which the application part is rotated;
FIG. 4 is a side view showing a tube type container having three balls as rotary bodies;
FIG. 5 is a front view showing a container according to the present invention having rollers as rotary bodies;
FIG. 6 is a view showing various shapes of a single rotary body (e.g. a ball) in the case in which different kinds of metals are disposed in the rotary body; and
FIG. 7 is a view showing various shapes of a single rotary body (e.g. a roller) in the case in which different kinds of metals are disposed in the rotary body.

### [Best Mode]

Now, exemplary embodiments of a container having an application part according to the present invention will be described in detail with reference to the accompanying drawings. It is obvious, however, that the present invention is not limited by the following embodiments.

The structure of a composition reception part of the container according to the present invention is not particularly limited by those described herein. For example, the composition reception part may be configured so as to have a tube type structure, a big mouth type structure, a small mouth type structure, a pump type structure, or a vacuum type structure.

The shape of a composition application part of the container according to the present invention is not particularly limited by those described herein. Any composition application part may be used, as long as the composition application part is provided with a rotary body. For example, the composition application part may be formed so as to have a circular shape, an oval shape, a square shape, a rectangular shape, a trapezoidal shape, or a gourd shape, when viewed from the front.

The composition application part may be directly connected to the composition reception part. Alternatively, the composition application part may be connected to the composition reception part via a guide part, which guides a composition, such as a medicine or cosmetic, from the composition reception part to the composition application part. In addition, the composition application part may be configured so as to be stationary or freely rotatable. From the aspect of convenience, however, the composition application part is preferably provided with a so-called stopper for holding and fixing the composition application part at a specific position in the state in which the composition application part is still rotatable. The composition application part may be elongated when viewed from the front, and may be coupled to the composition reception part so as to be freely rotatable. In the case in which the composition application part has both a rotation function and a stopper function, the composition application part may be conveniently used as follows. In the case in which the composition application part is disposed in the vertical direction when viewed from the front, it is possible to easily apply the composition on nasolabial folds. On the other hand, in the case in which the composition application part is disposed in the horizontal direction when viewed from the front, it is possible to easily apply the composition on wide surfaces of the face, such as the cheeks. The stopper function may be achieved by adopting a well-known structure, such as an undercut structure, depending on the shape of the composition application part.

In the present invention, a ball or a cylindrical roller may be used as the rotary body. The ball or the roller may be selectively used. Alternatively, both the ball and the roller may be used together. The size of the rotary body is not particularly restricted. For ease of application, however, the rotary body preferably has a diameter ranging from 0.3 to 1.5 cm, more specifically from 0.7 to 0.9 cm.

The rotary body that may be used in the present invention is made of metals having a potential difference therebetween caused by the ionization of a composition received in the composition reception part and/or a liquid at the region at which the rotary body is used such that a galvanic current is generated when the rotary body is used. For example, the rotary body may be made of a single body (i.e. a single metal) having different kinds of metals, such as gold, silver, copper, platinum, zinc, titanium, rhodium, palladium, iridium, ruthenium, osmium, iron, aluminum, lithium, and stainless steel, an alloy of different metals, or different kinds of plated metals. The material for the rotary body is not particularly restricted, as long as different kinds of metals are provided during the rotation of the rotary body. Furthermore, in the case in which different kinds of metals are included in the rotary body, the rotary body may be made of a nonmetal material.

Since the magnitude of a galvanic current is increased as the potential difference between the different kinds of metals is increased, different kinds of metals that are capable of achieving the objects of the present invention may be selected.

The number of rotary bodies that can be used in the present invention is not particularly restricted.

In the case in which a single rotary body is used, it is necessary for different kinds of metals to be included in the rotary body in order to generate a galvanic current when the rotary body is used. In this case, for example, when a ball is used as the rotary body, one hemisphere of the ball may be plated with gold, and the other hemisphere of the ball may be plated with chrome. Alternatively, the entirety of the ball may be plated with gold, and portions of the ball having dot shapes may be plated with chrome, like a soccer ball. On the other hand, in the case in which a cylindrical roller is used as the rotary body, opposite end portions of the roller may be plated with gold, and the middle portion of the roller therebetween may be plated with platinum. In this case, if different kinds of metals are in contact with each other, current is directly generated between the different kinds of metals. As a result, a galvanic current may not be generated in the region at which the composition is desired to be applied. For this reason, an insulator is preferably disposed between the different kinds of metals.

In the case in which two or more rotary bodies are used in the present invention, the arrangement of the rotary bodies is not particularly restricted. It is sufficient to dispose the rotary bodies at the position at which a galvanic current can be generated when the rotary bodies are used. In the case in which the rotary bodies are disposed so as to be close to the position at which a galvanic current can be generated, the galvanic current effects may be expected. Of course, the rotary bodies may be disposed so as to be far from the position at which galvanic current can be generated.

A rotary body holding part that can be used in the present invention is not particularly restricted, as long as the rotary body holding part is cable of holding the rotary body such that the rotary body can be successfully rotated without being separated from the rotary body holding part during application, and any well-known method may be used. In the case in which a ball is used as the rotary body, methods disclosed in, for example, Japanese Patent Application Publication No. M 50-156295 and Japanese Patent Application Publication No. 2007-152125 may be used. On the other hand, in the case in which a roller is used as the rotary body, methods disclosed in, for example, Japanese Patent Application Publication No. 2011-189342 and Japanese Patent Application Publication No. 2012-210959 may be used.

Hereinafter, concrete embodiments of the present invention will be described. However, the present invention is not limited to the following embodiments.

### <First embodiment>

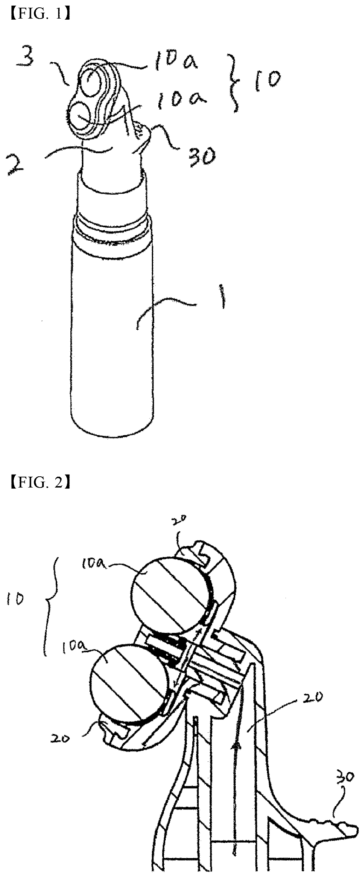

FIG. 1 is a view showing a dispenser type container according to a first embodiment of the present invention.

Referring to FIG. 1, a composition application part 3 is connected to a composition reception part 1 for receiving a medicine or cosmetic via a guide part 2. That is, the container shown in the figure is a so-called vacuum pump type container. The composition application part 3 is provided with two balls 10a as rotary bodies 10. One of the balls 10a is made of stainless steel, and the other of the balls 10a is plated with gold. The balls 10a are rotatably held by a rotary body holding part 20. The composition application part 3 is provided with a rib, through which the composition application part 3 is rotatable. The rib is configured to stop the composition application part 3 in two directions, e.g. in the vertical direction and in the horizontal direction, when viewed from the front.

The container shown in FIG. 1 may be used as follows.

When a user pushes a handle 30 provided at the container, a composition received in the composition reception part 1 is pushed upwards to the guide part 2 as the result of a well-known pumping action. The upwardly pushed composition reaches the rear of the composition application part 3, and then contacts the balls 10a. As the user appropriately moves the composition application part 3 on his/her face in the state in which the balls 10a are in contact with his/her face, the composition is applied to his/her face in response to the rotation of the balls 10a. In the case in which the user wishes to intensively apply the composition on facial wrinkles, such as nasolabial folds, the user may use the composition application part 3 in the state in which the composition application part 3 is fixed in the vertical direction when viewed from the front. Consequently, it is possible to intensively apply the composition on the nasolabial folds. On the other hand, in the case in which the user wishes to apply the composition over wide surfaces of the face, such as the cheeks, the user may use the composition application part 3 in the state in which the composition application part 3 is fixed in the horizontal direction when viewed from the front. Consequently, it is possible to easily apply the composition over the wide surfaces of the face.

### <Second embodiment

### Tube type container having three balls

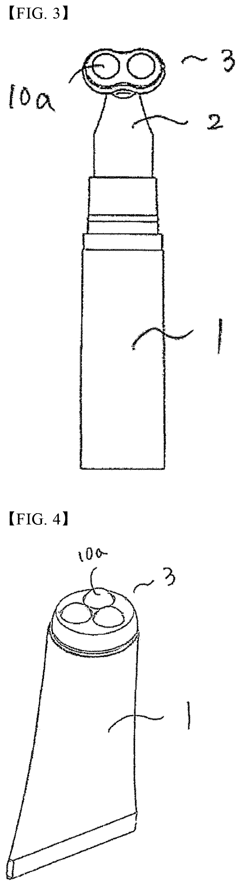

FIG. 4 is a view showing a tube type container having three balls as rotary bodies. In this embodiment, a composition application part 3 is directly connected to a composition reception part 1. That is, no guide part 2 is provided.

The composition application part 3 is formed to have a circular shape when viewed from the front. Three balls 10a, serving as rotary bodies 10, are mounted in the composition application part 3 such that the balls are spaced apart from each other by a predetermined distance. In this embodiment, the balls 10a are arranged at the same interval. Alternatively, two of the balls 10a may be arranged so as to be close to each other, and the other of the balls 10a may be arranged so as to be spaced apart from the two balls arranged so as to be close to each other. In this embodiment, one of the balls 10a is plated with gold, and two of the balls 10a are plated with silver.

The container shown in FIG. 4 may be used as follows. When the user lightly holds the tube type container, a composition is pushed to the mouth of the composition reception part. Subsequently, the composition moves to the rear of the composition application part 3, and then comes into contact with the balls 10a. As the user moves the composition application part 3 on his/her face in the state in which the balls 10a are in contact with his/her face, the composition is applied to his/her face in response to the rotation of the balls 10a.

### <Third embodiment>

### Container having two rollers

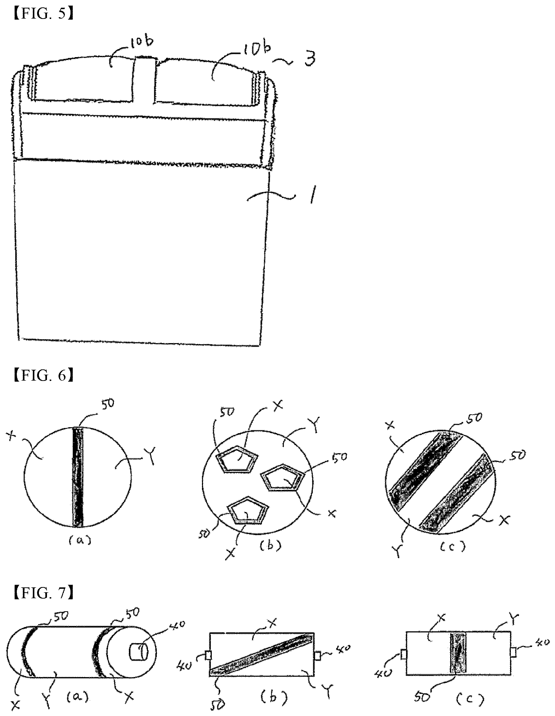

FIG. 5 is a view showing a container having two rollers 10b, which are used in place of the balls in the first embodiment or in the second embodiment. This embodiment is identical in principle to that of the first embodiment or the second embodiment, even though the rollers 10b are used in place of the balls.

### <Fourth embodiment>

### Ball type rotary body

FIG. 6 is a view showing various shapes of a single ball, which is used as a rotary body, in the case in which different kinds of metals are disposed in the ball. In FIG. 6, black portions indicate insulators 50.

### (a) Hemisphere type ball

Two hemispheres of the ball are made of different kinds of metals.

For example, an insulator 50 is disposed between the hemispheres of the ball. In this case, the hemisphere X may be plated with chrome, and the hemisphere Y may be plated with gold.

### (b) Soccer ball type ball

A base metal portion is provided with metal portions, each of which has a dot shape and a kind of metal that is different from the metal of the base metal portion.

For example, the base metal portion Y may be made of stainless steel, and metal portions X may be plated with silver. The metal portions X are separated from the base metal portion Y by insulators 50.

### (c) Stripe type ball

Different kinds of metals are arranged in a stripe fashion.

For example, the ball may be partitioned into several portions by insulators 50. The portions X may be plated with gold, and the portion Y may be plated with aluminum.

FIG. 7 is a view showing various shapes of a single roller, which is used as a rotary body, in the case in which different kinds of metals are disposed in the roller. In FIG. 7, the black parts indicate insulators 50.

### (a) Sandwich type roller

Opposite end portions and the middle portion of the roller are plated with different metals.

For example, the opposite end portions X and the middle portion Y of the roller may be partitioned from each other by insulators 50. The end portions X of the roller may be plated with platinum, and the middle portion Y of the roller may be plated with aluminum.

### (b) Stripe type roller

Different kinds of metals are arranged in a stripe fashion, in the same manner as the ball shown in FIG. 6.

For example, the roller may be partitioned into two portions by an insulator 50. The portion X may be made of stainless steel, and the portion Y may be plated with chrome.

### (c) Separate type roller

Two halves of the roller are made of different kinds of metals, in the same manner as in the ball shown in FIG. 6.

For example, one half X of the roller may be plated with copper, and the other half Y of the roller may be plated with gold.

FIGS. 6 and 7 are given only for illustrative purposes. In the case in which a plurality of portions X is provided in the same figure, the portions X may not be made of the same kind of metal. The portions X may be made of different kinds of metals, as long as the object of the present invention is achievable.

### [Industrial Applicability]

According to the present invention, the container is capable of generating a galvanic current through a simple structure and of providing high cosmetic effects. Consequently, the container according to the present invention is capable of being used as a tool for applying various kinds of medicines and cosmetics.

### <Description of Reference Symbols>

1: Composition reception part
2: Guide part
3: Composition application part
10: Rotary body
10a: Balls
10b: Rollers
20: Rotary body holding part
30: Handle
40: Shaft

## Claims

1. A container comprising:
a composition reception part; and
a composition application part, wherein
the composition application part is provided with two or more rotary bodies, and
each of the rotary bodies is made of metals having a potential difference therebetween caused by ionization of a composition received in the composition reception part and/or a liquid in a region at which each of the rotary bodies is used.

2. A container comprising:
a composition reception part; and
a composition application part, wherein
the composition application part is provided with a rotary body, and
the rotary body has two or more metal portions having a potential difference therebetween caused by ionization of a composition received in the composition reception part and/or a liquid in a region at which the rotary body is used.

3. The container according to claim 1 or 2, wherein each of the metals or each of the metal portions is selected from a group consisting of a single body having different kinds of metals, an alloy, and different kinds of plated metals.

4. The container according to any one of claims 1 to 3, wherein the composition application part is rotatable.
